# EUROPEAN PATENT SPECIFICATION

(11) **EP 2 788 016 B1**
(45) Date of publication and mention of the grant of the patent: **01.02.2017**
(21) Application number: 12805653.8
(22) Date of filing: 06.12.2012
(51) Int. Cl.: A61K 38/44, A61P 17/00

(54) **COMPOSITIONS FOR PREVENTING OR TREATING ADVERSE REACTIONS OF EGFR INHIBITION**
ZUSAMMENSETZUNGEN ZUR PRÄVENTION ODER BEHANDLUNG VON NEBENWIRKUNGEN EINER EGFR-HEMMUNG
COMPOSITIONS POUR PRÉVENIR OU TRAITER DES RÉACTIONS DÉFAVORABLES DE L'INHIBITION D'EGFR

(30) Priority: 06.12.2011 EP 11192223
(43) Date of publication of application: 15.10.2014
(73) Proprietor: SCARLETRED Holding GmbH, 1030 Wien (AT)
(72) Inventor: LOIBNER, Hans, A-1230 Vienna (AT); SCHNIDAR, Harald, A-1150 Vienna (AT)
(74) Representative: Sonn & Partner Patentanwälte
(86) International application number: PCT/EP2012/074645
(87) International publication number: WO 2013/083695

(56) References cited:
- US-B1- 6 312 720
- MELOSKY B ET AL: "Management of skin rash during EGFR-targeted monoclonal antibody treatment for gastrointestinal malignancies: Canadian recommendations.", CURRENT ONCOLOGY, vol. 16, no. 1, January 2009 (2009-01), pages 14-24, XP002674583, TORONTO, ONT. ISSN: 1198-0052
- TEJWANI AJAY ET AL: "Increased risk of high-grade dermatologic toxicities with radiation plus epidermal growth factor receptor inhibitor therapy.", CANCER, vol. 115, no. 6, 15 March 2009 (2009-03-15), pages 1286-1299, XP002674584, ISSN: 0008-543X

## Description

The present invention relates to pharmaceutical compositions comprising recombinant human superoxide dismutase (rhSOD) for preventing or treating one or more adverse reactions, especially acne-like skin rash, caused by treatment with an EGFR inhibitor, such as an anti-EGFR antibody or an EGFR tyrosine kinase inhibitor, in a subject in need thereof.

Epidermal Growth Factor Receptor (EGFR) mutation or overexpression has been identified to be the cause of many different types of cancer like carcinomas and glioblastomas, which are tumors of epithelial and glial origin, respectively. Patients suffering from such tumors can be systemically treated with EGFR inhibitors, which, however, may cause severe adverse inflammatory side effects, especially in the skin and the gut, often leading to abortion of therapy.

Inhibition of EGFR can be achieved through two classes of therapeutics: Tyrosine Kinase Inhibitors (TKIs) and anti-EGFR antibodies.

As described for example in Heidary et al., 2008; and Robert et al., 2005, treatment with EGFR inhibitors is associated with inflammatory cutaneous side effects in more than 50% of the patients, which may result in premature treatment termination. Importantly, the intensity of the inflammatory side effects in the skin positively correlates with the anti-cancer treatment success (Perez-Soler et al., 2004). Patients displaying the strongest cutaneous side effects are also those showing better anti-tumor response and longer survival after anti-EGFR treatment.

The most common cutaneous side effect of patients receiving anti-EGFR therapies is sterile, non-infectious folliculitis, an acne-like papulopustular rash, also called acne-like skin rash (ALSR), which occurs early after start of treatment. Lesions frequently occur in facial areas and are accompanied by a dermal infiltrate of a mixed population of blood leukocytes like neutrophils, eosinophils, but also T-cells. The second most frequent side effects are hair modifications like abnormal scalp, face hair, and eyelash growth. Furthermore, patients treated with EGFR inhibitors very frequently develop gastrointestinal disturbances like diarrhea and nausea.

The above described adverse reactions of EGFR therapy very often lead to drug discontinuation or dose reduction, impairs the quality of life of the patients and moreover, puts patients at risk of superinfection.

However, there are no standard treatments or guidelines yet to manage the adverse reactions caused by EGFR inhibition. Treatment of skin related adverse reactions is currently limited to topical antibiotics, general skin care and hygiene recommendations. The use of other treatment options, such as topical steroids, is controversial because of secondary side effects. Topical retinoids are not recommended because of the further increase in skin dryness and peeling, and acne medications are not as effective as steroids or antibiotics. Systemic steroids for treatment of severe skin or gastrointestinal adverse reactions are problematic, since they may interfere with EGFR inhibition.

Melosky et al. (Curr. Oncol. 16 (2009), 14-24) disclose recommendations for the management of skin rash during EGFR-targeted mAb treatment for gastrointestinal malignancies. The efficacy of EGFRI therapy using monoclonal antibodies such as cetuximab and panitumumab is confined to patients with non-mutated (wildtype) KRAS (Melosky et al. 2009).

Tejwani et al. (Cancer 115 (2009), 1286-1299) report an increased risk for high-grade radiation dermatitis, rash and mucositis with EGFR inhibitor treatment plus radiation.

Thus, there is a high need for novel therapeutic strategies to manage adverse reactions in patients treated with EGFR inhibitors. Therefore, it is the aim of the present invention to provide pharmaceutical compositions for preventing or treating one or more adverse reactions caused by treatment with an EGFR inhibitor.

Accordingly, the invention provides pharmaceutical compositions comprising recombinant human superoxide dismutase (rhSOD) for use in preventing or treating one or more adverse reactions caused by treatment with an EGFR inhibitor in a subject, wherein the one or more adverse reaction of EGFR inhibition is selected from dermatologic toxicity reactions, rash, acne-like skin rash, paronychia, fissuring, hyperpigmentation, hypopigmentation, xerosis (dry skin), pruritus, desquamation, mucositis, stomatitis, hypersensitivity reactions, alopecia, trichomegalia, hypertrichosis, hand-foot skin reaction, delayed hair growth, diffuse hair loss, and/or changes in hair structure.

The pathomechanism underlying the inflammation following treatment with EGFR inhibitors is only poorly understood. In particular the role and interplay of the various resident and infiltrating immune cell populations has so far not been investigated. In healthy skin, specific immune cell populations are located in the distinct skin layers constituting the skin associated lymphoid tissue. Human and mouse epidermis (and the epidermis of other species as well) contain Langerhans cells (LCs), which are a subtype of dendritic cells (DCs) present in stratified epithelia. The immune cell populations in the dermis are much more heterogeneous. Healthy dermis contains DCs, CD4+ and CD8+ T-cells, γδ T-cells and natural killer T-cells (NKT), macrophages and mast cells. A specialized subtype of inflammatory DCs (IDCs) appears in inflamed human dermis like in psoriasis and is called TIP-DCs ("TNFα and iNOS producing DCs"). In different inflammatory skin disorders typical Th-cell subtypes can be identified by key cytokines they are producing. More recently, Th-17 cells have been described to be important in chronic inflammatory disorders. However, cutaneous T-cells are also important for preventing inflammation under normal conditions. An example is the induction of regulatory T-cells (Tregs) by UV-B irradiation mediated via LCs, which highlights the complicated interactions among skin cell populations (Yoshiki et al., 2010).

Skin mast cells are specially equipped to sense various danger signals induced by skin damage or infection. They express a broad range of pattern recognition receptors like TLRs, complement- and Fc-receptors as well as cytokine receptors. Besides pathogens, danger signals derived from epithelia like cathelicidin LL37, which is regulated by EGFR, may attract and activate mast cells (Lande et al., 2007; Schiemann et al., 2009). Neutrophil/granulocytes are recruited to the skin in response to a variety of triggers like wounding, bacterial infection or during chronic inflammatory skin diseases like psoriasis or systemic lupus erythematodes. In keratinocytes some of the signals responsible for chemoattraction, like IL-8, CCL-2 or GM-CSF, may be regulated by EGFR (Mascia et al., 2003; Roupe et al., 2010). In wound healing and infectious diseases neutrophils are essential for reconstitution (Lin et al., 2011 b), whereas their large repertoire of proinflammatory mediators, like reactive oxygen species (ROS), myeloperoxidase or interleukin-17a, may cause autoimmune like diseases under certain conditions (Lin et al., 2011 a; Villanueva et al., 2011).

Some proinflammatory mediators like CCL-2/MCP-1, CCL-5/RANTES and CXCL-10/IP-10, which may recruit dendritic cells (DCs), T-cells and neutrophils, are down regulated in response to EGFR stimulation. Importantly, production of these chemokines induced by cytokines like TNFα is enhanced when EGFR signaling is inhibited (Mascia et al., 2003), providing a possible explanation for the increase in neutrophils, which are also one of the main source for ROS in the skin of EGFR inhibitor-treated patients. How the different immune cell types and superoxide production contribute and collaborate to induce the acne-like skin rash (ALSR) phenotypes observed in patients treated with EGFR inhibitors is currently still poorly understood.

The pathomechanism underlying the gastrointestinal side effects of treatment with EGFR inhibitors is also only poorly understood. Mice lacking the EGFR ligand TGFα showed increased susceptibility to DSS induced colitis (Egger et al., 1997). EGFR ligands are increased in response to DSS triggered mucosal injury and this response depends on TLR4 signaling (Hsu et al., 2010). It is hypothesized that the TLR4 mediated increase in EGFR ligand expression is important for mucosal cell proliferation and repair in response to tissue damage. Analysis of bone marrow chimeric mice has revealed that for the increased release of EGFR ligands the TLR-adapter molecule MyD88 is needed in non-hematopoietic cells, presumably mucosal epithelial cells, and that mice harboring an attenuated version of the EGFR (waved2, velvet mutants) are more susceptible for DSS induced colitis (Brandl et al., 2010; Egger et al., 2000). Interestingly, EGFR signaling also seems to be involved in the interaction with gut microbiota. For example, the probiotic protein p40 produced by *Lactobacillus rhamnosus GG* protects intestinal epithelial cells from apoptosis in an EGFR/Akt dependent manner (Yan et al., 2011). Treatment of mice with established DSS-induced colitis by p40 dramatically improved mucosal barrier function and reduced disease symptoms highlighting the importance of EGFR signaling in gut homeostasis.

The approach employed in the present invention is unique as it employs very specific and highly suitable mouse models (tissue-specific EGFR knock-out mice), which develop a very similar phenotype as cancer patients subjected to anti-EGFR targeted therapies. By using the tissue-specific EGFR knock-out method, it is possible to generate mice lacking the EGFR specifically in the skin and/or gut. Mice lacking the EGFR specifically in the skin develop a severe skin inflammation resembling ALSR. Thus, such mice are versatile tools to investigate the mechanism underlying the phenotypes induced by EGFR inhibitors, as well as for testing compounds aimed at preventing and/or treating the adverse reactions of EGFR inhibitor treatment. In the present invention, superoxide dismutase (SOD) has been tested in these mouse models.

Superoxide dismutases (SODs) are a class of enzymes that catalyze the dismutation of the superoxide anion into oxygen and hydrogen peroxide. As such, they are an important antioxidant defense in nearly all cells exposed to oxygen. In humans (as in all other mammals and most chordates), three forms of superoxide dismutase are present. SOD1 is located in the cytoplasm, SOD2 in the mitochondria, and SOD3 is extracellular. The first is a dimer (consists of two units), whereas the others are tetramers (four subunits). SOD1 and SOD3 contain copper and zinc, whereas SOD2, the mitochondrial enzyme, has manganese in its reactive center. Native human Cu/Zn-SOD1 is a homo-dimeric molecule (32kDa). It is essential for protecting human cells from harmful levels of oxidative stress by counteracting the development of ROS. It specifically reacts with the superoxide anion (•O₂⁻) radicals, which can act as protective armor against bacterial infections. Though based on their intrinsic oxidative reactivity on biological tissue they are also capable to induce undesired inflammatory responses and carcinogenic modifications on proteins, lipids, DNA and RNA. Increased formation of •O₂⁻ can be induced either by external environmental factors, like sources of irradiation or mutagenic chemicals or naturally by specialized human immune cells such as macrophages and neutrophils upon the process of respiratory burst.

In the present invention, SOD has been shown in the above described mouse models to positively influence several T-cell related parameters affected by EGFR inhibition and thus, provides evidence for the use of SOD to prevent or treat adverse reactions of EGFR inhibition. Accordingly, the present invention provides pharmaceutical compositions comprising recombinant human superoxide dismutase (rhSOD) for use in preventing or treating one or more adverse reactions caused by treatment with an EGFR inhibitor in a subject in need thereof, wherein the one or more adverse reaction of EGFR inhibition is selected from dermatologic toxicity reactions, rash, acne-like skin rash, paronychia, fissuring, hyperpigmentation, hypopigmentation, xerosis (dry skin), pruritus, desquamation, mucositis, stomatitis, hypersensitivity reactions, alopecia, trichomegalia, hypertrichosis, hand- foot skin reaction, delayed hair growth, diffuse hair loss, and/or changes in hair structure.

In an embodiment of the present invention, the rhSOD is a recombinant human Cu/Zn-SOD, most preferably a recombinant human SOD1. The rhSOD may be produced in an E. coli expression system, preferably by use of a batch/fed batch fermentation process, and preferably in a volume of 100L. After separation by centrifugation, purification may be performed by ammonium sulfate precipitation and one or more chromatographic steps, preferably three different chromatographic steps, and most preferably based on different Sepharose^{®} resins. Afterwards, the rhSOD may be sterile filtrated and optional-ly formulated. The rhSOD may be stored in physiological PBS, preferably at a concentration of 30±10 mg/ml.

The rhSOD may be used as free rhSOD, as described e.g. in Tsao et al, 1991; Land et al. 1994; Davis et al. 1997; or Davis et al. 2004. In a preferred embodiment, the pharmacokinetic characteristics of the rhSOD may be improved by use of a drug delivery system. In another preferred embodiment, the rhSOD may be modified, for example, pegylated or lecithinized (Igarashi et al., 1992).

In an especially preferred embodiment, the rhSOD is encapsulated in liposomes. Suitable liposomal rhSOD compositions are described for example in WO 96/14083 and can be produced as described in WO 2002/036257.

In a preferred embodiment, the liposomes are composed of three different lipids. Suitable lipids that can be used according to the present invention, especially for topical application of the pharmaceutical composition, are described, for example, in Braun et al. 2006. In a preferred embodiment, the liposomes according to the invention comprise cholesterol and 1,2-dipalmitoyl-*sn*-glycero-3-phosphocholine (DPPC). In a further preferred embodiment, the third lipid comprised in the liposomes according to the invention is selected from the group comprising 1,2-dimyristoyl-*sn*-glycero-3-[phosphor-rac-(1-glycerol)](sodium salt) (DMPG), or 1,2-dipalmitoyl-phosphatidylglycerol (DPPG), or egg phosphatidyl-glycerol (EPG), or stearylamine (SA). In an especially preferred embodiment, DPPC and cholesterol are present in the molar ratio of 7:2. In another preferred embodiment, cholesterol and the third lipid (DMPG, DPPG, EPG, or stearylamine) are present in a ratio of 2:1. In an embodiment, the liposomes are composed of 1,2-dipalmitoyl-*sn*-glycero-3-phosphocholine (DPPC), cholesterol and 1,2-dimyristoyl-*sn*-glycero-3-[phosphor-rac-(1-glycerol)](sodium salt) (DMPG) in the molar ratio of 7:2:1.

In yet another preferred embodiment, the liposomes are unilamellar. In still another embodiment, the liposomes have an average diameter of less than 500 nm, or less than 350 nm, or less than 300 nm, preferably 220 ± 50 nm, more preferably less than 250 nm. In an even more preferred embodiment, the liposomes have an average diameter of 200 ± 10 nm.

In an embodiment, the pharmaceutical composition comprises APN201. In an embodiment, the pharmaceutical composition is APN201. APN201 is a liposomal rhSOD formulation comprising 1.6 mg/ml liposomal rhSOD, 1 mg/ml methylparaben, 5 mg/ml Carbopol 981 NF, 1.44 mg/ml sodium phosphate dibasic dihydrate, 0.2 mg/ml potassium chloride, 0.2 mg/ml potassium phosphate (monobasic), and 8 mg/ml sodium chloride. High quality water is added ad 1 ml. The liposomes of APN201 are composed of 1,2-dipalmitoyl-sn-glycero-3-phosphocholine (DPPC), cholesterol and 1,2-dimyristoylsn-glycero-3-[phosphor-rac-(1-glycerol)](sodium salt) (DMPG) in the molar ratio of 7:2:1 and have an average diameter of 220 ± 50 nm. The liposomal rhSOD and methods for production are also described in WO1996/14083 and WO2002/036257. The pharmaceutical composition according to the invention may further comprise hyaluronic acid. The hyaluronic acid (also called hyaluronan) may be present in the pharmaceutical composition in a concentration of 0.1 to 1% by weight based on the pharmaceutical composition. The hyaluronic acid may have a molecular mass of 250 kDa to 1000 kDa, preferably 250 kDa to 500 kDa. In one embodiment, the hyaluronic acid is a non-animal stabilized hyaluronic acid (NASHA). In one embodiment, the NASHA is Synocrom^{®} and/or Durolane^{®}. Suitable hyaluronan gels are described e.g. in Brown et al., J Invest Dermatol. 1999 Nov; 113(5):740-6.

In one embodiment, the pharmaceutical composition according to the invention is for enteral administration, preferably oral administration and may be administered, for example, in the form of enteric-coated capsules or tablets. In another embodiment, the pharmaceutical composition according to the invention is for parenteral administration, such as, e.g., intravenous, intradermal or subcutaneous, or for rectal administration, e.g. as suppository.

In another embodiment, the pharmaceutical composition according to the invention is for topical application and may be administered in the form of an emulsion, a suspension, solution, lotion, ointment or gel, or by spraying with a spray device.

In one embodiment of the present invention, the pharmaceutical composition is applied to the skin area in liquid form by spraying on the affected skin area from a spray can or spray bottle. This avoids direct contact of the affected skin with the fingers or another aid for application, and thus reduces the danger of an additional infection.

Preferably, the pharmaceutical composition according to the invention comprises 0.5-10 mg rhSOD per ml of the pharmaceutical composition, preferably 1.6 ± 0.5 mg/ml.

In one embodiment of the pharmaceutical composition according to the invention the rhSOD is present in a concentration of 0.01 to 5% by weight based on the pharmaceutical composition.

The pharmaceutical compositions according to the invention most preferably comprise, depending on the use and method of application, rhSOD in a concentration of ≥0.01% by weight, in particular of 0.05 to 5% by weight, based on the prepared, ready-to-use pharmaceutical composition. For topical applications, an amount of 0.01 to 2 mg rhSOD/cm² skin area or body surface area may be used. On the other hand, oral or parenteral applications are advantageously carried out with rhSOD doses of 0.5 to 50 mg rhSOD/kg body weight, the dose advantageously being adjusted to 0.5 to 10 mg rhSOD/kg per day for repeated SOD doses during a therapy.

The pharmaceutical composition according to the invention, especially if intended for topical application, may further comprise at least one low-fat or fat-free excipient, preferably an organic or inorganic hydrogel, and/or hyaluronic acid. In an especially preferred embodiment of the invention, the pharmaceutical composition comprises at least one polyacrylic acid or carbomer, such as e.g. Carbopol, most preferably Carbopol 981 NF. The Carbopol may be present in a concentration of 0.5-2% by weight of the final formulation, most preferably 0.5% by weight.

Further substances, such as water, buffering agents, preservatives and/or skin care factors, may also be present in the pharmaceutical composition according to the present invention. Preferred buffering agents are selected from the group comprising sodium phosphate dibasic dihydrate, potassium chloride, potassium phosphate (monobasic), and sodium chloride. A preferred preservative is methylparaben.

In an especially preferred embodiment of the invention, the pharmaceutical composition comprises 1.6 mg/ml liposomal rhSOD, 1 mg/ml methylparaben, 5 mg/ml Carbopol 981 NF, 1.44 mg/ml sodium phosphate dibasic dihydrate, 0.2 mg/ml potassium chloride, 0.2 mg/ml potassium phosphate (monobasic), and 8 mg/ml sodium chloride. High quality water is added ad 1 ml.

The pharmaceutical composition according to the invention is preferably administered 1, 2, 3, 4, or 5 times per day.

In a particular aspect of the present invention, the pharmaceutical composition according to the invention is administered before, during, and/or after the treatment period with at least one EGFR inhibitor. In one embodiment, the treatment period with the pharmaceutical composition according to the invention is started together with the EGFR inhibition therapy. In another embodiment, the treatment period with the pharmaceutical composition according to the invention is started prior to the beginning of the EGFR inhibition therapy. In still another embodiment, the pharmaceutical composition according to the present invention is administered during the treatment period with the at least one EGFR inhibitor. In yet another preferred embodiment, the pharmaceutical composition according to the present invention is administered after termination of the treatment period with the at least one EGFR inhibitor. In an embodiment, the treatment with the pharmaceutical composition according to the present invention is continued after termination of the treatment period with the at least one EGFR inhibitor. In a most preferred embodiment, the pharmaceutical composition according to the invention is used before, during, and after the treatment period with the at least one EGFR inhibitor.

In one embodiment, the EGFR inhibitor is a reversible or irreversible EGR inhibitor. In one embodiment, the EGFR inhibitor is a tyrosine kinase inhibitor (TKI) and/or an anti-EGFR antibody. In an embodiment, the TKI is a low molecular weight molecule which works on the intracellular domain of EGFR by stopping ligand-induced receptor auto-phosphorylation. In an embodiment, the anti-EGFR antibody is a monoclonal antibody targeting the extracellular domain of EGFR.

In a preferred embodiment of the invention, the EGFR inhibitor is an anti-EGFR antibody, including but not limited to Cetuximab (Erbitux^{®}) and Panitumumab (Vectibix^{®}), Matuzumab (also referred to as EMD 72000), Necitumumab, and Zalutumumab.

In another preferred embodiment, the EGFR inhibitor is a tyrosine kinase inhibitor, especially a low molecular weight EGFR tyrosine kinase inhibitor. Such low molecular weight EGFR tyrosine kinase inhibitors include, for example, Erlotinib (Tarceva®), Gefitinib (Iressa®), Lapatinib (Tyverb®/Tykerb®), Canertinib, Pelitinib (also referred to as EKB-569), Afatinib, and Vandetanib. In an embodiment, the invention relates to a pharmaceutical composition comprising rhSOD for treating one or more adverse reactions caused by treatment with an EGFR inhibitor in a subject in need thereof, wherein the one or more adverse reaction of EGFR inhibition is selected from dermatologic toxicity reactions, rash, acne-like skin rash, paronychia, fissuring, hyperpigmentation, hypopigmentation, xerosis (dry skin), pruritus, desquamation, mucositis, stomatitis, hypersensitivity reactions, alopecia, trichomegalia, hypertrichosis, hand- foot skin reaction, delayed hair growth, diffuse hair loss, and/or changes in hair structure.

In one embodiment of the invention, the adverse reaction of EGFR inhibition is a dermatologic toxicity reaction, i.e. an adverse reaction affecting skin, nail, mucosa, or hair. In an especially preferred embodiment of the invention, the adverse reaction of EGFR inhibition is rash, especially acne-like skin rash (ALSR). Acne-like skin rash, as used herein, shall include synonyms, such as acneiform rash, acne-like papulopustular rash, papulopustular eruptions, acneiform eruptions, pimple-like rash, pimple-like eruptions, and folliculitis. ALSR begins as facial erythema followed by papules (small bumps) and pustules (small pockets of pus) mainly over the face and upper trunk. Unlike true acne, the pustules are sterile (they contain no bacteria). Accordingly, the term ALSR as used herein shall further include the different phases of ALSR, such as facial erythema, papules and pustules. Acne-like skin rash may be characterized by or associated with abnormal epithelial cell turnover, follicular occlusion, follicular rupture, premature differentiation of basal keratinocytes, growth arrest of basal keratinocytes, release of one or more inflammatory mediators by keratinocytes (e.g. cytokines and/or chemokines), keratinocyte apoptosis, and a disrupted epidermal barrier function. The acne-like skin rash may be graded according to Common Toxicity Criteria Common or Terminology Criteria of Adverse Events (CTCAE), e.g. the Common Terminology Criteria for Adverse Events (CTCAE) version 3.0, DCTD, NCI, NIH, DHHS, March 31, 2003, published August 9, 2006 (http://ctep.cancer.gov/protocoldevelopment electron-ic applications/docs/ctcaev33.pdf), the CTC according to the National Cancer Institute (NCI) (e.g. NCI CTC criteria version 2.0, 3.0, or 4.0), the CTC of the World Health Organization (WHO), the CTC of the South West Oncology Group (SWOG), or according to the MASCC EGFR Inhibitor Skin Toxicity Tool (MESTT) developed by the Skin Toxicity Study Group of the Multinational Association of Supportive Care in Cancer (www.mascc.org), see also Supportive Care in Cancer, Volume 19, Number 8, 1079-1095, DOI: 10.1007/s00520-011-1197-6.

In one embodiment, the adverse reaction of EGFR inhibition to be prevented or treated according to the invention is an adverse reaction, e.g. acne-like skin rash, of grade 1, 2, and/or 3 according to the CTCs or CTCAE mentioned above (e.g. the CTC according to the NCI). In one embodiment, the adverse reaction of EGFR inhibition to be prevented or treated according to the invention is an adverse reaction, e.g. acne-like skin rash, of grade 2 and/or 3 according to the CTCs or CTCAE mentioned above (e.g. the CTC according to the NCI). In one embodiment, the adverse reaction of EGFR inhibition to be prevented or treated according to the invention is an adverse reaction, e.g. acne-like skin rash, of grade 2 according to the CTCs or CTCAE mentioned above (e.g. the CTC according to the NCI). In one embodiment, the adverse reaction of EGFR inhibition to be prevented or treated according to the invention is an adverse reaction, e.g. acne-like skin rash, of grade 3 according to the CTCs or CTCAE mentioned above (e.g. the CTC according to the NCI).

In one embodiment, the one or more adverse reaction to be prevented or treated according to the present invention is characterized by an increase in hematopoetic cells (e.g. CD45 positive cells) in cells of the epidermis and/or the dermis. In one embodiment, the one or more adverse reaction to be prevented or treated according to the present invention is characterized by an increased expression level of neutrophil-attracting chemokines (e.g. CCL2 and/or CCL5) in keratinocytes. In one embodiment, the one or more adverse reaction to be prevented or treated according to the present invention is characterized by an increased level of a specific subset of CD45 positive hematopoetic cells, namely CD11 b+Gr-1 high cells (neutrophils), in cells of the epidermis and/or the dermis. In one embodiment, the one or more adverse reaction to be prevented or treated according to the present invention is characterized by a decrease in memory T cells (e.g. CD44 positive cells). In one embodiment, the one or more adverse reaction to be prevented or treated according to the present invention is characterized by a decrease in TCR gamma delta (TCRγδ) - CD3e high and/or in TCR gamma delta (TCRγδ) - CD3e low cells.

The adverse reactions to be prevented or treated according to the present invention include paronychia, fissuring, hyperpigmentation, hypopigmentation, xerosis (dry skin), pruritus, desquamation, mucositis, stomatitis, hypersensitivity reactions, alopecia, trichomegalia, hypertrichosis, hand-foot skin reaction (synonyms: hand-foot syndrome, acral erythema, palmar plantar erythema, hand-to-foot syndrome, Burgdorf reaction, palmar-plantar erythrodysesthesia, palmar plantar dysaestesia, palmar plantar erythroderma, toxic erythema of the palms and soles), delayed hair growth, diffuse hair loss, and/or changes in hair structure, such as e.g. brittleness (for further description of such dermatologic adverse events see e.g. Cancer Management: 14th edition; Dermatologic Adverse Events Associated With Targeted Therapies; Mario E. Lacouture, December 2011; Munk and El-Helou 2012, Dermatology Rounds Vol 7, Issue 3, Targeted Molecular Therapies - What are the Associated Cutaneous Risks; and Saif MW et al. 2010, Journal of the Pancreas 11(2):176-182).

The present invention is further illustrated by the following examples and figures without being restricted thereto.

### Figures

Fig. 1 shows the phenotype of EGFR^{dEP} mutants (indicated by an asterisk) and control littermates at postnatal day 2 (P2; A), P10 (B) and P20 (C). Fig. 1D and E show that EGFR^{dEP-ER} mice progressively lose hair and develop inflammation within 2-4 weeks after treatment start.
Fig. 2 shows the inflammatory infiltrate in the skin of EGFR^{dEP} mice. The quantitative analysis of the number of hematopoietic cells infiltrating into the epidermis (A) and dermis (B) by flow cytometry shows massive infiltration of inflammatory cells in EGFR mutant skin. Hematopoietic cells were stained by anti-mouse CD45 antibody; n = 3.
Fig. 3 shows that EGFR^{dEP} keratinocytes produce neutrophil-attracting chemokines. The quantitative real-time PCR analysis shows increased levels of CCL-2 and CCL-5, whereas GM-CSF production is slightly reduced in cultured keratinocytes from EGFR^{dEP} compared to littermate control mice; n = 3.
Fig. 4 shows that neutrophils infiltrate into the dermis. The analysis of the infiltrate into the dermis of 3-4 month old EGFR^{dEP} mice is depicted compared to littermate controls and shows increased numbers of CD11b⁺ Gr-1^{high} neutrophils; n = 3.
Fig. 5 shows one representative example of the altered activation of the epidermal sub population of gamma-delta T-cells (γδ -T-cells) in EGFR^{dEP-ER} knock out mice; n = 2.
Fig. 6 shows one representative example of the reduced expression of CD44 surface marker in γδ -T-cells - CD3e high and low expressing cells upon EGFR^{dEP-ER} knock out compared to littermate control. Note the reverting effect of APN201 (liposomal rhSOD) treatment on CD44 surface expression in EGFR^{dEP-ER} (red line/ bars) versus the Placebo (blue line / bars); n = 2.
Fig. 7 shows the effect of rhSOD on Langerhans cells of the skin (cell extract of dermis and epidermis) in both mouse models. Mice were treated with APN201 for 16 days, n = 3 except for the two EGFRdEP groups, where n was 1 for Placebo treatment and 2 for SOD treatment. The EGFR^{dEP-ER} mice have been treated with TX as described above for 8 weeks prior to start of the APN201 treatment. Both placebo treated knockout models show an increased level of Langerhans cells in the skin, whereas SOD treated knockouts showed significantly lower levels resembling the situation in EGFR wild-type mice.

### Examples

### Example 1: Mouse animal model

Mice carrying a loxP-flanked (floxed) allele of the EGFR were generated as described in Natarajan et al. 2007. These mice allow deleting the EGFR in any given cell-type by breeding them to transgenic mice expressing the Cre recombinase, which recognizes and recombines loxP-flanked DNA sequences, in a tissue-specific manner. For epidermal-specific deletion of the EGFR two transgenic lines have been used. In the K5-Cre transgenic line (Tarutani et al., 1997) Cre recombinase is active from embryonic day E14.5 on in basal cells of stratified epithelia like in the skin or in the esophagus. Thus, EGFR is knocked out in skin already at birth. To be able to knock out the EGFR in young or adult mice, K5-CreER mice can be used (Metzger and Chambon, 2001). In these mice, Cre recombinase is fused to a mutated estrogen receptor (ER), which prevents the Cre recombinase from shuttling into the nucleus unless the estrogen analogon 4-OH tamoxifen binds to the ER. Thus, the lack of EGFR can be induced upon systemic tamoxifen treatment in young or adult mice. For initiation, 1 mg of tamoxifen per 25 g body weight (Sigma; sunflower seed oil/ethanol mixture (10:1) at 10 mg/ml) was injected intraperitoneally (i.p.) for five consecutive days plus 2 days off dose per cycle (1 cycle = 1 week). To maintain knock-out keratinocytes after said initiation, 1 mg of tamoxifen per 25 g body weight tamoxifen was injected i.p. twice a week. Mice have been treated with tamoxifen for at least 8 weeks prior to analysis of untreated mice or prior to the start of APN201 treatment.

For conditional deletion of the EGFR in gut epithelium both these approaches, constitutive and inducible deletion, can be applied by breeding EGFR floxed mice to VilCre and Vil-CreERCreER transgenic mice, respectively (el Marjou et al., 2004; Madison et al., 2002). With Vil-Cre/CreER mice, the EGFR can be deleted in all crypt cells and villus epithelial cells of the small and large intestine.

Mice lacking the EGFR specifically in the skin or gut are versatile tools to investigate the function of EGFR in the respective tissues. The striking overlap of the symptoms of mice lacking the EGFR in these tissues to cancer patients systemically treated with EGFR inhibitors makes these mice ideal tools to investigate on the one hand the mechanism underlying the phenotypes induced by EGFR inhibitors, on the other hand they provide an ideal tool for testing compounds aimed at reducing the side effects of inhibitor treatment.

### Example 2: Generation and characterization of mice lacking EGFR in the epidermis and gut

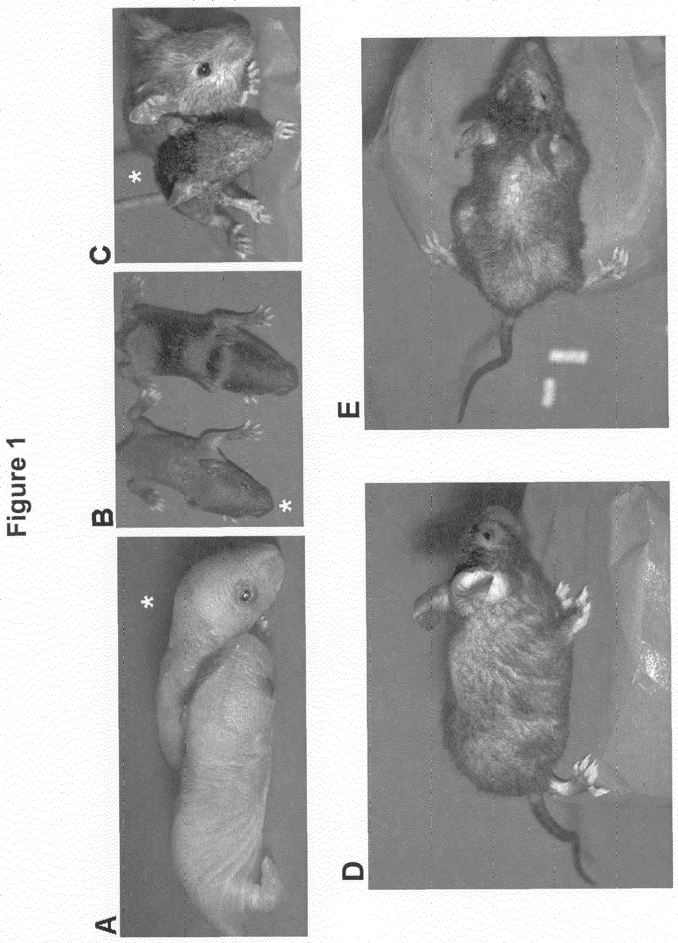

EGFR mutant mice show various epithelial defects, fail to develop a hairy coat and die within the first weeks of life. Moreover, conditional knockout mice lacking the EGFR specifically in stratified epithelia (EGFR^{dEP} mice) with a K5-Cre transgenic line (Tarutani et al., 1997) develop a phenotype similar to EGFR^{-/-} mice. EGFR^{dEP} mutants are born with open eyes, fail to develop a hairy coat and only few mice survive longer than one month after birth (Fig. 1A-C). Those mice surviving the first 4 weeks can get up to 6 month old, are bald and show macroscopic and microscopic signs of skin inflammation. Moreover, by 4 weeks of age these mice are significantly smaller than the controls. For further method desciptions, e.g. the isolation of dermal and epidermal cell suspensions and flow cytometric analysis, see Example 4 and Holcmann M, Stoitner P et. al; J Immunol. 2009 Jul 15;183(2):1133-43.

Mice in which deletion of the EGFR can be induced upon systemic or topical administration of the estrogen-analogue Tamoxifen (TX) have been generated by breeding EGFR^{fl/fl} mice to the inducible K5-CreER transgenic line (Metzger and Chambon, 2001). Thereby, it is possible to delete EGFR from the epidermis at any given time point desired, e.g. in young or adult mice. EGFR^{dEP}-ER mice lose hair comparably to EGFR^{dEP} mice, display skin inflammation but do not show premature death (Fig. 1 D and E).

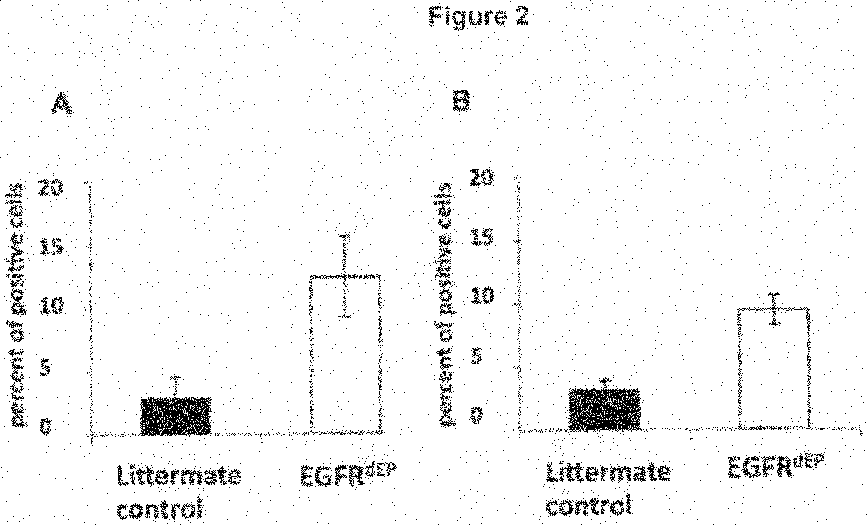

Mice lacking EGFR in the epidermis develop skin inflammation similar to what observed in cancer patients treated with EGFR inhibitors. Analysis of skin samples shows massive infiltration of inflammatory cells in EGFR mutant skin (Fig. 2). Overall the percentage of infiltrating hematopoietic cells is increased almost 3 fold in epidermis (Fig. 2A) and dermis (Fig. 2B) of EGFR^{dEP} mice.

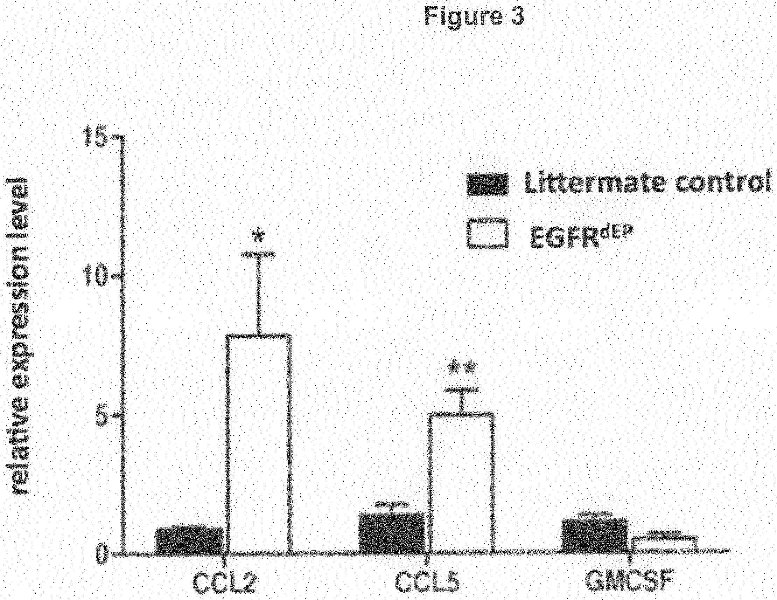

It has previously been reported that human keratinocytes treated with EGFR inhibitors up regulate the chemokines CCL2/MCP-1 and CCL5/RANTES, whereas GM-CSF is down regulated (Mascia et al., 2010; Mascia et al., 2003). Also in keratinocytes of EGFR^{dEP} mice we found increased levels of CCL-2 and CCL-5 whereas GM-CSF production was slightly reduced (Fig. 3) demonstrating that the mouse phenotypes mimic the situation seen in EGFR inhibitor treated human samples.

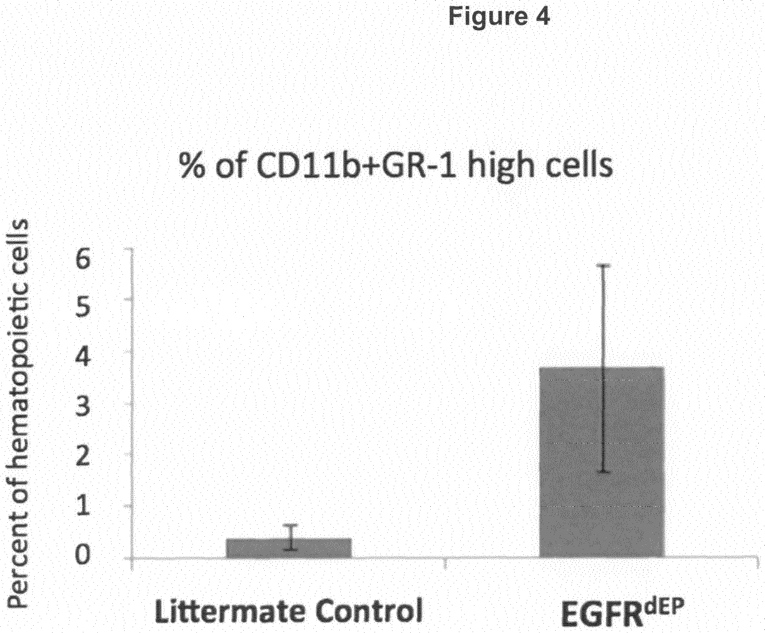

CCL-2 and CCL-5 are chemokines responsible for the attraction of neutrophils and T-cells. We next investigated whether we could detect these cells in the infiltrate into the skin of EGFR^{dEP} mice. In analogy to the neutrophil-dependent folliculitis described in affected skin of patients treated with EGFR inhibitors we found, besides DCs, increased numbers of CD11 b+ Gr-1 high neutrophils and T-cells infiltrating into the dermis whereas the number of macrophages was not affected (Fig. 4 and additional data not shown).

Due to the high overlap with the human skin pathologies developing after EGFR inhibitor treatment, EGFR^{dEP}, EGFR^{dEP-ER} mice reflect the severe adverse drug event "Acne Like Skin Rash (ALSR)" which is described upon treatment with Tarceva^{®} (Erlotinib), Erbitux^{®} (Cetuximab), Vectibix^{®} (Panitumumab), or other EGFR inhibitors in human cancer patients and thus represent valuable tools to study the effect of SOD treatment on the inflammatory phenotype caused by the lack of EGFR signaling.

The above described TX-inducible K5-CreER transgenic mice have been used to eliminate EGFR specifically in the epidermis. As, described above, this mouse model develops skin inflammation upon EGFR deletion and reflects the severe adverse drug event "Acne Like Skin Rash (ALSR)" in human cancer patients receiving EGFR inhibitors. This mouse model has been employed to test for the anti inflammatory potential of a hydrogel formulation of liposomal packaged rhSOD (APN201).

### Example 3: Effects of SOD in the tissue-specific EGFR knockout mouse model

Mice have been topically treated with APN201 (liposomal rhSOD) and placebo (vehicle control) according to the treatment plan given in Table 1 and the impact on the inflammatory phenotype has been investigated. At the end of the treatment period, the mice were sacrificed and FACS analysis was conducted on freshly isolated skin tissue material, which was stained for the expression of T-lymphocyte-, and Neutrophil cell surface markers.

**Table 1:**

| **Parameter** | **Description** |
|---|---|
| Experiment Design | Topical drug/placebo application |
| Species | Mouse (C57BL/6 background) |
| | Tamoxifen inducible conditional skin specific EGFR knockout mouse (EGFR^{dEP-ER}) Skin specific stable EGFR knock out mouse (EGFR^{dEP}) |
| Randomization | 10 animals (5m/5f) APN201 10 animals (5m/5f) Placebo (vehicle only) control |
| Application | 1 area of 1 cm x 4 cm (4 cm²) APN201 or placebo Untreated local control of 1 cm x 1 cm (1 cm²) All areas are on the mouse backskin and have been shaved 24 hours prior to treatment |
| Dose | 0.05 ml / 5 cm² (0.08 mg rhSOD / cm²) corresponding to 0.01 ml of the pharmaceutical composition / cm² (0.0016 mg rhSOD / cm²) |
| Treatment | 1 dose per day on 5 consecutive days plus 2 days off dose per cycle (1 cycle = 1 week) |
| Duration | 2, 4 or 5 cycles (= 10 treatments within 2 weeks, 20 treatments within 4 weeks; 25 treatments with in 5 weeks) |
| Evaluation | Visual and by FACS Analysis |

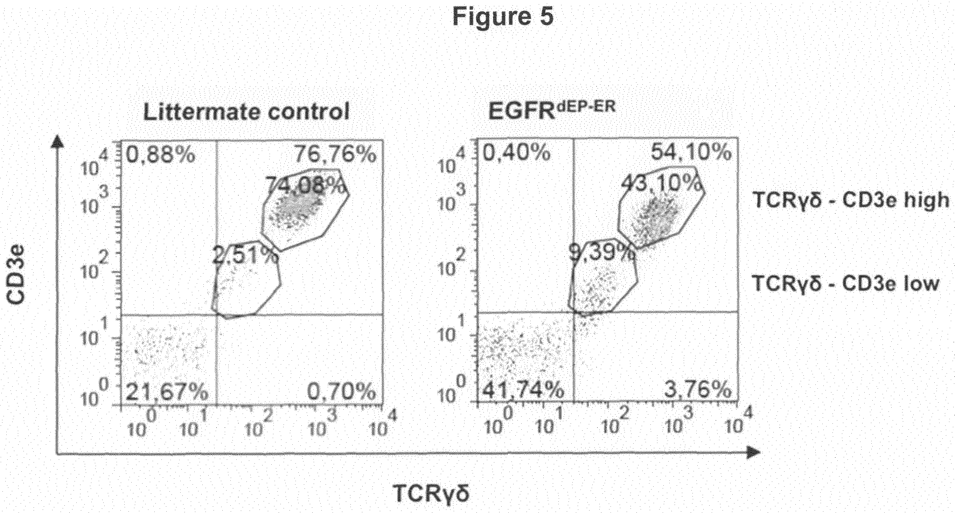

As given in detail in Fig. 5, an altered activation of TCRγδ-CD3e positive T-cells in the epidermal compartment of EGFR^{dEP-ER} knockouts could be detected. The relative number of positive TCRγδ-CD3e cells (high and low population) is significantly reduced from 74% in wild type control mice down to 43% in the EGFR^{dEP-ER} knockouts. This surprising result is a clear sign for TCRγδ-CD3e receptor internalization, and thus, increased T-cell activation in mice upon skin specific inhibition of the EGFR signal.

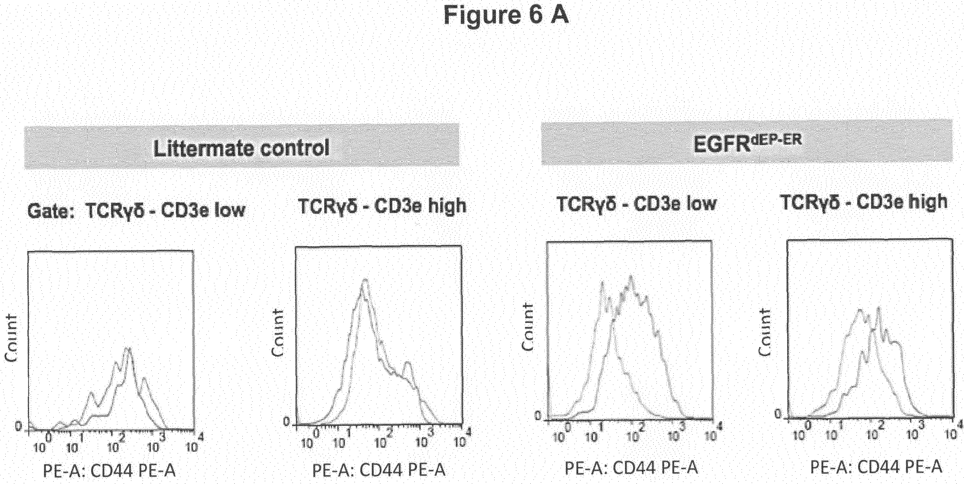

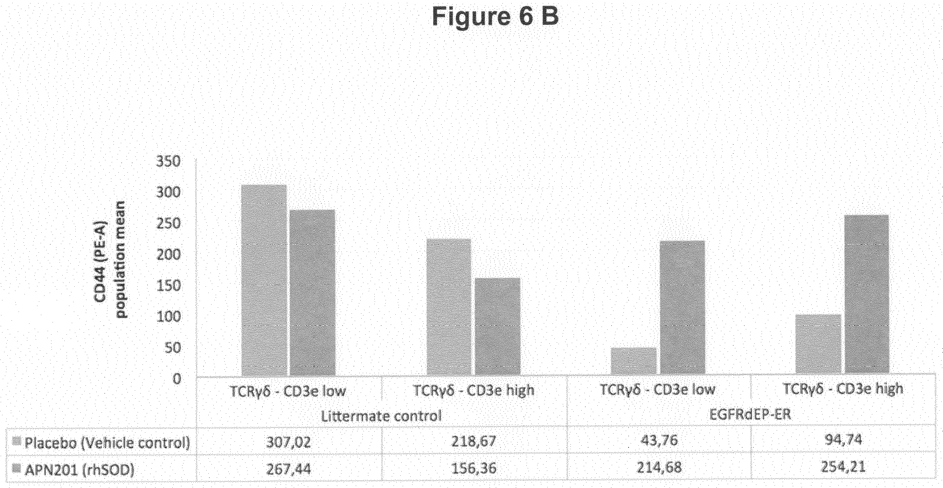

Furthermore, Fig. 6 shows that positive TCRγδ-CD3e cells (high and low population) is massively down regulated within the CD44 positive cell population in the EGFR^{dEP-ER} knockout. CD44 is a prominent cell surface marker well known for its anchorage-function by interaction with Hyaluronic Acid (HA), as well as for its dual role in the control of inflammation. Surprisingly, down-regulation of can be efficiently counteracted by topical APN201 treatment. Already known for its anti-inflammatory role in lung injury, CD44 is involved in the resolution of inflammation by increasing the activation of TGF-β1, leading to the removal of apoptotic neutrophils and both high molecular mass and fragmented HA. In contrast, loss of CD44 delays or prevents this resolution (Teder et al., 2002). For the first time, the liposomal encapsulated rhSOD formulation (APN201) has been proven to re-activate or increase the positive TCRγδ-CD3e cells (high and low population) within the CD44 positive cell population which were down-regulated by the absence of the EGFR.

The present animal data provides evidence to use APN201 or other rhSODs as novel drugs in the treatment of EGFR targeted cancer therapy induced adverse reactions.

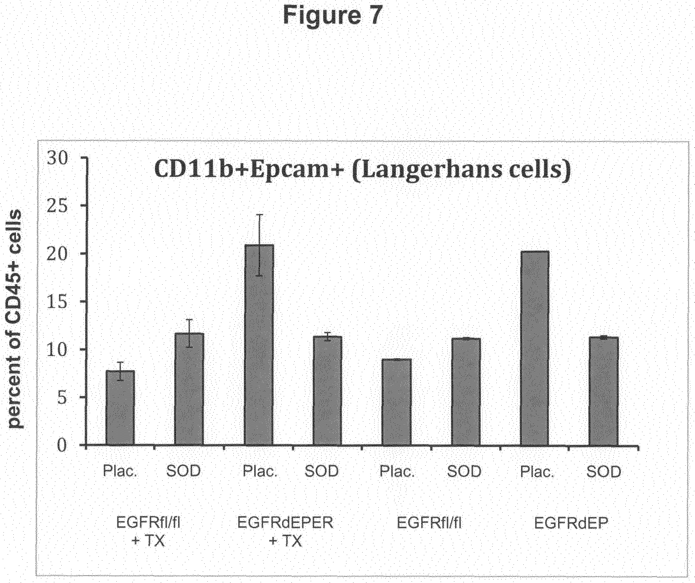

### Example 4: Detailed methods for Experiments 1 to 3 and Figures 1 to 7

### Preparation of murine backskin cell suspension

Mice were killed by cervical dislocation. After isolation of murine backskin subcutaneous fat was scraped off and sample was immediately incubated in ice-cold PBS. Subsequently, PBS was replaced by 0.8% trypsine and samples were incubated for 45 minutes at 37°C. Epidermis and dermis were separated and incubated with DNAse-Medium for 30 minutes at 37°C with agitation (600rpm). Samples were separately transferred into a 6-well and 5ml of ice-cold Würzburg buffer (5% FCS, 5 mM EDTA, 4 ng/ml DNAsel, PBS) were added. Single cell suspensions were prepared using a glass pipette and thereafter filtered through a cell strainer. After centrifugation at 1100rpm for 7 minutes at 4°C supernatant was discarded and the cell pellet was resuspended in 1ml of ice-cold PBS.

To monitor an influence of liposomal rhSOD on the immune cell compartment EGFRdEP ER mice were topically treated with APN201 or vehicle as control (empty liposomes as described above, 1 mg/ml methylparaben, 5 mg/ml Carbopol 981 NF, 1.44 mg/ml sodium phosphate dibasic dihydrate, 0.2 mg/ml potassium chloride, 0.2 mg/ml potassium phosphate (monobasic), 8 mg/ml sodium chloride, and high quality water ad 1 ml) for 2 or 5 weeks as described above and percentage of immune cell and their activation state was analyzed by flow cytometric analysis. Cell suspensions from epidermis and dermis were isolated as described above and filtered through a 70µm nylon mesh, counted and stained with mAbs for 30 min in PBS + 5% FCS at 4°C after blocking with anti CD16 / CD32 antibody (Biolegend). The following mAbs were used:
anti-CD3ε-PE (clone145-2C11),
anti-CD45-APC-Cy7 (clone 30F11),
anti CD44 PE (clone IM7),
anti TCRgd-APC (clone GL-3),
anti-CD11b-FITC (clone M1/70.15),
and anti EpCAM (clone G8.8), all from Biolegend.
Dead cells were excluded by adding 7AAD (Sigma) at a final concentration of 1µg/mL after the last washing step.

### RNA isolation

All centrifugation steps were performed with a pre-cooled centrifuge. After isolation of epidermal and dermal cell suspension, samples were centrifuged for 6 minutes at 400g. Pellets were resuspended in 1ml of TRIzol Reagent (Invitrogen; Carlsbad, CA, USA) and RNA was isolated according to the manufacturer's protocol. After addition of 200µl chloroform samples were vortexed for 15 seconds and incubated at room temperature for 3 minutes. Phase separation was achieved by centrifugation at 12,000g for 15 minutes. The aqueous phase was transferred into a new tube and incubated with 500µl of isopropyl alcohol for 10 minutes at room temperature. After a 10 minutes centrifugation step the supernatant was discarded and the pellet was washed with 1ml of 75% ethanol and centrifuged for 10 minutes at 7,500g. The pellet was dried at 37°C and resolved in nuclease-free water. Total RNA concentration was measured using a Nanodrop spectrophotometer (NanoDrop ND-1000; NanoDrop Technologies, Wilmington, DE).

### cDNA synthesis

Reverse transcription was performed using SuperScript II Reverse Transcriptase (Invitrogen; Carlsbad, CA, USA) according to the manufacturer's protocol for random primers with 1µg of total RNA.
Quantitative real-time PCR. Transcription levels of respective genes were quantified using SYBR Green PCR Master Mix (Applied Biosystems; Carlsbad, CA, USA) and an ABI Prism 7000 thermal cycler using following primers:

| | |
|---|---|
| CCL2-1 | AGG TCC CTG TCA TGC TTC TG |
| CCL2-2 | TCT GGA CCC ATT CCT TCT TG |
| | |
| CCL5-3 | ATATGGCTCGGACACCACTC |
| CCL5-4 | CCCACTTCTTCTCTGGGTTG |
| mGM-CSF-F | GGAGGATGTGGCTGCAGAAT |
| mGM-CSF-R | GGCTGTAGACCACAATGCCC |

After normalization to TATA-box binding protein (Tbp) relative expression levels were calculated using the 2^{-ΔΔCt} method.

### Statistical methods

All values are expressed as the mean and standard deviation of individual samples unless explicitly stated else. Samples were analyzed using the two tailed Student's t-test.

## Claims

1. A pharmaceutical composition comprising recombinant human superoxide dismutase (rhSOD) for use in preventing or treating one or more adverse reactions caused by treatment with an EGFR inhibitor in a subject, wherein the one or more adverse reaction of EGFR inhibition is selected from dermatologic toxicity reactions, rash, acne-like skin rash, paronychia, fissuring, hyperpigmentation, hypopigmentation, xerosis (dry skin), pruritus, desquamation, mucositis, stomatitis, hypersensitivity reactions, alopecia, trichomegalia, hypertrichosis, hand-foot skin reaction, delayed hair growth, diffuse hair loss, and/or changes in hair structure .

2. The pharmaceutical composition for use according to claim 1, wherein the rhSOD is a recombinant human Cu/Zn-SOD.

3. The pharmaceutical composition for use according to any of the preceding claims, wherein the rhSOD is recombinant human SOD1.

4. The pharmaceutical composition for use according to any of the preceding claims, wherein the rhSOD is encapsulated in liposomes.

5. The pharmaceutical composition for use according to claim 4, wherein the liposomes are unilamellar liposomes.

6. The pharmaceutical composition for use according to any one of claims 4 or 5, wherein the liposomes have an average diameter of less than 500 nm, or less than 350 nm, or less than 300, or less than 250 nm.

7. The pharmaceutical composition for use according to claim 6, wherein the liposomes have an average diameter of 220 ± 50 nm or 200 ± 10 nm.

8. The pharmaceutical composition for use according to any of the preceding claims, wherein the composition is for systemic administration.

9. The pharmaceutical composition for use according to any of the preceding claims, wherein the composition is for oral administration.

10. The pharmaceutical composition for use according to any of claims 1 to 7, wherein the composition is for topical application.

11. The pharmaceutical composition for use according to claim 10, wherein the composition is administered in the form of an emulsion, a suspension, solution, lotion, ointment or gel, or by spraying with a spray device.

12. The pharmaceutical composition for use according to any of the preceding claims, wherein the rhSOD is present in a concentration of 0.01 to 5% by weight based on the pharmaceutical composition.

13. The pharmaceutical composition for use according to any of the preceding claims, wherein the pharmaceutical composition comprises 0.5-10 mg rhSOD per g of the pharmaceutical composition.

14. The pharmaceutical composition for use according to any of the preceding claims, wherein the pharmaceutical composition comprises 1.6 ± 0.5 mg/g rhSOD per g of the pharmaceutical composition.

15. The pharmaceutical composition for use according to any of the preceding claims, wherein the rhSOD is used in a concentration of 0.01 to 2 mg rhSOD/cm² of skin area or body surface area for topical application, or in a concentration of 0.5-50 mg rhSOD/kg body weight for oral administration.

16. The pharmaceutical composition for use according to any of the preceding claims, wherein the pharmaceutical composition further comprises at least one low-fat or fat-free excipient.

17. The pharmaceutical composition for use according to claim 15, wherein the excipient is an organic or inorganic hydrogel, and/or hyaluronic acid.

18. The pharmaceutical composition for use according to any of the preceding claims, wherein the pharmaceutical composition is administered 1, 2, 3, 4, or 5 times per day.

19. The pharmaceutical composition for use according to any of the preceding claims, wherein the pharmaceutical composition according to the invention is administered before, during, and/or after the treatment period with the at least one EGFR inhibitor.

20. The pharmaceutical composition for use according any of the preceding claims, wherein the EGFR inhibitor is an anti-EGFR antibody or an EGFR tyrosine kinase inhibitor.

21. The pharmaceutical composition for use according to any of the preceding claims, wherein the adverse reaction of EGFR inhibition is acne-like skin rash.

## Patentansprüche

1. Pharmazeutische Zusammensetzung, umfassend rekombinante humane Superoxiddismutase (rhSOD), zur Verwendung in der Prävention oder Behandlung einer oder mehrerer durch die Behandlung mit einem EGFR-Inhibitor verursachter unerwünschter Wirkungen bei einem Patienten, wobei die ein oder mehreren unerwünschten Wirkungen der EGFR-Inhibition ausgewählt sind aus dermatologischen Toxizitätsreaktionen, Hautausschlag, Akne-artigem Hautausschlag, Paronychie, Rissbildung, Hyperpigmentation, Hypopigmentation, Xerose (trockene Haut), Juckreiz, Hautschuppung, Mukositis, Stomatitis, Überempfindlichkeitsreaktionen, Alopezie, Trichomegalie, Hypertrichose, Hand-Fuß-Hautreaktion, verzögertem Haarwachstum, diffusem Haarausfall und/oder Veränderungen in der Haarstruktur.

2. Pharmazeutische Zusammensetzung zur Verwendung gemäß Anspruch 1, wobei die rhSOD eine rekombinante humane Cu/Zn-SOD ist.

3. Pharmazeutische Zusammensetzung zur Verwendung gemäß einem der vorangehenden Ansprüche, wobei die rhSOD rekombinante humane SOD1 ist.

4. Pharmazeutische Zusammensetzung zur Verwendung gemäß einem der vorangehenden Ansprüche, wobei die rhSOD in Liposomen verkapselt ist.

5. Pharmazeutische Zusammensetzung zur Verwendung gemäß Anspruch 4, wobei die Liposomen unilamellare Liposomen sind.

6. Pharmazeutische Zusammensetzung zur Verwendung gemäß einem der Ansprüche 4 oder 5, wobei die Liposomen einen mittleren Durchmesser von weniger als 500 nm, oder weniger als 350 nm, oder weniger als 300 nm, oder weniger als 250 nm aufweisen.

7. Pharmazeutische Zusammensetzung zur Verwendung gemäß Anspruch 6, wobei die Liposomen einen mittleren Durchmesser von 220 ± 50 nm oder 200 ± 10 nm aufweisen.

8. Pharmazeutische Zusammensetzung zur Verwendung gemäß einem der vorangehenden Ansprüche, wobei die Zusammensetzung für die systemische Verabreichung ist.

9. Pharmazeutische Zusammensetzung zur Verwendung gemäß einem der vorangehenden Ansprüche, wobei die Zusammensetzung für die orale Verabreichung ist.

10. Pharmazeutische Zusammensetzung zur Verwendung gemäß einem der Ansprüche 1 bis 7, wobei die Zusammensetzung für die topische Applikation ist.

11. Pharmazeutische Zusammensetzung zur Verwendung gemäß Anspruch 10, wobei die Zusammensetzung in Form einer Emulsion, einer Suspension, Lösung, Lotion, Salbe oder Gel, oder durch Sprühen mit einer Sprühvorrichtung verabreicht wird.

12. Pharmazeutische Zusammensetzung zur Verwendung gemäß einem der vorangehenden Ansprüche, wobei die rhSOD in einer Konzentration von 0,01 bis 5 Gewichts-%, basierend auf der pharmazeutischen Zusammensetzung, vorliegt.

13. Pharmazeutische Zusammensetzung zur Verwendung gemäß einem der vorangehenden Ansprüche, wobei die pharmazeutische Zusammensetzung 0,5-10 mg rhSOD pro g der pharmazeutischen Zusammensetzung umfasst.

14. Pharmazeutische Zusammensetzung zur Verwendung gemäß einem der vorangehenden Ansprüche, wobei die pharmazeutische Zusammensetzung 1,6 ± 0,5 mg/g rhSOD pro g der pharmazeutischen Zusammensetzung umfasst.

15. Pharmazeutische Zusammensetzung zur Verwendung gemäß einem der vorangehenden Ansprüche, wobei die rhSOD in einer Konzentration von 0,01 bis 2 mg rhSOD/cm² der Hautfläche oder der Körperoberfläche für die topische Applikation oder in einer Konzentration von 0,5-50 mg rhSOD/kg Körpergewicht für die orale Verabreichung verwendet wird.

16. Pharmazeutische Zusammensetzung zur Verwendung gemäß einem der vorangehenden Ansprüche, wobei die pharmazeutische Zusammensetzung außerdem mindestens einen fettarmen oder fettfreien Exzipienten umfasst.

17. Pharmazeutische Zusammensetzung zur Verwendung gemäß Anspruch 15, wobei der Exzipient ein organisches oder anorganisches Hydrogel und/oder Hyaluronsäure ist.

18. Pharmazeutische Zusammensetzung zur Verwendung gemäß einem der vorangehenden Ansprüche, wobei die pharmazeutische Zusammensetzung 1, 2, 3, 4 oder 5 Mal pro Tag verabreicht wird.

19. Pharmazeutische Zusammensetzung zur Verwendung gemäß einem der vorangehenden Ansprüche, wobei die pharmazeutische Zusammensetzung gemäß der Erfindung vor, während und/oder nach dem Behandlungzeitraum mit dem mindestens einen EGFR-Inhibitor verabreicht wird.

20. Pharmazeutische Zusammensetzung zur Verwendung gemäß einem der vorangehenden Ansprüche, wobei der EGFR-Inhibitor ein Anti-EGFR-Antikörper oder ein EGFR-Tyrosinkinase-Inhibitor ist.

21. Pharmazeutische Zusammensetzung zur Verwendung gemäß einem der vorangehenden Ansprüche, wobei die unerwünschte Reaktion der EGFR-Inhibition Akneartiger Hautausschlag ist.

## Revendications

1. Une composition pharmaceutique comprenant une superoxyde dismutase humaine recombinante (rhSOD) pour prévenir ou traiter une ou plusieurs effets indésirables provoqués chez un patient par un traitement par un inhibiteur de l'EGFR, dans laquelle les un ou plusieurs effets indésirables de l'inhibition de EGFR sont choisies dans le groupe formé par des réactions de toxicité dermatologique, éruption (rash), éruption cutanée (rash cutané) de type acné, tourniole, fissures, hyperpigmentation, hypopigmentation, xérosis (peau sèche), prurit, desquamation, mucosite, stomatite, réactions d'hypersensibilité, alopécie, trichomégalie, hypertrichose, réaction cutanée mains-pieds; croissance capillaire retardée, perte diffuse de cheveux et/ou modifications de leur structure.

2. La composition pharmaceutique pour une utilisation selon la revendication 1, dans laquelle rhSOD est du Cu/Zn-SOD humain recombinant.

3. La composition pharmaceutique pour une utilisation selon une quelconque des revendications précédentes, dans laquelle rhSOD est du SOD1 recombinant humain.

4. La composition pharmaceutique pour une utilisation selon l'une quelconque des revendications précédentes, dans laquelle le rhSOD est encapsulé dans des liposomes.

5. La composition pharmaceutique pour une utilisation selon la revendications 4, dans laquelle les liposomes sont des liposomes unilamellaires.

6. La composition pharmaceutique pour une utilisation selon l'une quelconque des revendications 4 ou 5, dans laquelle les liposomes ont un diamètre moyen inférieur à 500 nm, ou inférieure à 350 nm, ou inférieure à 300, ou inférieure à 250 nm.

7. La composition pharmaceutique pour une utilisation selon la revendication 6, dans laquelle les liposomes ont un diamètre moyen de 220 ± 50 nm ou de 200 ± 10 nm.

8. La composition pharmaceutique pour une utilisation selon l'une quelconque des revendications précédentes, dans laquelle la composition est destinée à une administration systémique

9. La composition pharmaceutique pour une utilisation selon l'une quelconque des revendications précédentes, dans laquelle la composition est destinée à une administration par voie orale.

10. La composition pharmaceutique pour une utilisation selon l'une quelconque des revendications 1 à 7, dans laquelle la composition est destinée à une application topique.

11. La composition pharmaceutique pour une utilisation selon la revendication 10, dans laquelle la composition est administrée sous forme d'une émulsion, d'une suspension, d'une solution, d'une lotion, d'une pommade ou d'un gel, ou par pulvérisation au moyen d'un dispositif de pulvérisation.

12. La composition pharmaceutique pour une utilisation selon l'une quelconque des revendications précédentes, dans laquelle rhSOD est présent en une concentration de 0,01 à 5% en poids par rapport au poids de la composition pharmaceutique.

13. La composition pharmaceutique pour une utilisation selon l'une quelconque des revendications précédentes, laquelle composition pharmaceutique comprend de 0,5 à 10% mg en poids de rhSOD par gramme de la composition pharmaceutique.

14. La composition pharmaceutique pour une utilisation selon l'une quelconque des revendications précédentes, laquelle composition pharmaceutique comprend 1,6 ± 0.5mg de rhSOD par gramme de la composition pharmaceutique.

15. La composition pharmaceutique pour une utilisation selon l'une quelconque des revendications précédentes, dans laquelle rhSOD est utilisé dans une concentration de 0,01 à 2 mg de rhSOD/cm² de la surface de peau ou de la zone de surface du corps dans le cas d'une application topique ou à une concentration de 0,5 à 50 mg de rhSOD/kg de poids corporel dans le cas d'une administration par voie orale.

16. La composition pharmaceutique pour une utilisation selon l'une quelconque des revendications précédentes, dans laquelle la composition pharmaceutique comprend en outre au moins un excipient à faible teneur en matière grasse ou exempt de matière grasse.

17. La composition pharmaceutique pour une utilisation selon la revendication 15, dans laquelle l'excipient est un hydrogel organique ou inorganique et/ou de l'acide hyaluronique.

18. La composition pharmaceutique pour une utilisation selon l'une quelconque des revendications précédentes, dans laquelle la composition pharmaceutique est administrée 1, 2, 3, 4 ou 5 fois par jour.

19. La composition pharmaceutique pour une utilisation selon l'une quelconque des revendications précédentes, dans laquelle la composition pharmaceutique selon l'invention est administrée avant, pendant et/ou après la période de traitement avec au moins ledit inhibiteur de EGFR.

20. La composition pharmaceutique pour une utilisation selon l'une quelconque des revendications précédentes, dans laquelle l'inhibiteur d'EGFR est un anticorps anti-EGFR ou un inhibiteur de l'EGFR tyrosine kinase.

21. La composition pharmaceutique pour une utilisation selon l'une quelconque des revendications précédentes, dans laquelle l'effet indésirable de l'inhibition de l'EGFR est une éruption cutanée telle l'acné.


### SEQUENCE LISTING

<110> APEIRON BIOLOGICS AG
<120> Compositions for preventing or treating adverse reactions of EGFR inhibition
<130> R 63262
<150> EP 11192223.3
   <151> 2011-12-06
<160> 6
<170> PatentIn version 3.5
<210> 1
   <211> 20
   <212> DNA
   <213> Artificial Sequence
<220>
   <223> PCR Primer
<400> 1
   aggtccctgt catgcttctg 20
<210> 2
   <211> 20
   <212> DNA
   <213> Artificial Sequence
<220>
   <223> PCR Primer
<400> 2
   tctggaccca ttccttcttg 20
<210> 3
   <211> 20
   <212> DNA
   <213> Artificial Sequence
<220>
   <223> PCR Primer
<400> 3
   atatggctcg gacaccactc 20
<210> 4
   <211> 20
   <212> DNA
   <213> Artificial Sequence
<220>
   <223> PCR Primer
<400> 4
   cccacttctt ctctgggttg 20
<210> 5
   <211> 20
   <212> DNA
   <213> Artificial Sequence
<220>
   <223> PCR Primer
<400> 5
   ggaggatgtg gctgcagaat 20
<210> 6
   <211> 20
   <212> DNA
   <213> Artificial Sequence
<220>
   <223> PCR Primer
<400> 6
   ggctgtagac cacaatgccc 20